# EUROPEAN PATENT SPECIFICATION

(11) **EP 1 117 351 B1**
(45) Date of publication and mention of the grant of the patent: **10.08.2005**
(21) Application number: 99950019.2
(22) Date of filing: 29.09.1999
(51) Int. Cl.: A61F 2/06, A61L 31/00, A61K 51/12

(54) **DRUG DELIVERY SYSTEMS**
MEDIKAMENTENZUFUHRSYSTEME
SYSTEMES D'ADMINISTRATION DE MEDICAMENTS

(30) Priority: 29.09.1998 US 162547
(43) Date of publication of application: 25.07.2001
(73) Proprietor: C.R. Bard Inc., Murray Hill, NJ 07974 (US)
(72) Inventor: GAMBALE, Richard, A., Tyngsboro, MA 01879 (US)
(74) Representative: HOFFMANN - EITLE
(86) International application number: PCT/US1999/022640
(87) International publication number: WO 2000/018331

(56) References cited:
- EP-A- 0 830 873
- WO-A-97/38730
- WO-A-97/42910
- WO-A-98/23228
- WO-A-98/29148
- DE-A- 19 703 482
- US-A- 5 562 922

## Description

The invention relates to systems for the localized delivery of drugs, and in particular, to systems for treating arterial and vascular disease, and in particular for treating coronary conditions and cardiovascular disease.

### Background of the Invention

Numerous medical conditions exist in which it is desirable to treat a localized area of disease in a body tissue. Pharmacological interventions aimed at the area of local disease can be administered systemically or directly. Medication that is administered systemically is generally distributed through the vascular system, gaining access thereto either by intravenous or intra-arterial infusion, by intramuscular or subcutaneous injection, or by gastrointestinal absorption. However, systemic administrations of drugs have systemic effects: drugs administered systemically affect all body tissues. To have an adequate effect on a localized area of pathology, the drug may need to be administered at levels that are damaging to other tissues. Untoward side-effects or actual toxicity can result from systemic exposure to certain drugs, despite favorable local efficacy. Correlatively, a safe level of systemic exposure may not provide enough local concentration of the pharmacological agent to treat the localized condition. In addition, blood levels of drugs may vary during the process of systemic drug transport, making total dosage delivered to the diseased area variable. Direct delivery of a medication to an internal treatment area would minimize systemic effect while maximizing therapeutic benefit. A locally effective means of administering a pharmaceutical agent could permit high doses to the diseased tissue while avoiding the side effects or toxicity of systemic exposure.

The treatment of malignant tumors provides an example of a medical condition where direct delivery would be desirable. Most chemotherapeutic agents for treating malignancies are administered systemically, through intravenous or intra-arterial infusion. Although the drugs are intended to affect the neoplasm preferentially, nonetheless they circulate in the bloodstream and have effects on many of the tissues they thereby encounter. These systemic effects limit the dosage of the drug that can be safely administered and thus limit the drug's overall antineoplastic effect. Delivery systems have been designed to restrict the systemic diffusion of the chemotherapeutic agent, but there remains a need in the art for an apparatus that will permit local and high-dose administration of these powerful medications.

Similarly, intravascular pathology is commonly found in discrete areas within blood vessels. These lesions are treated interventionally through balloon dilatation of a region of the artery that has been narrowed by atherosclerosis, a technique known as balloon angioplasty that is well-described in the prior art. However, when this technique is applied to the coronary arteries, in a procedure known as percutaneous transluminal coronary angioplasty (PTCA), the treated vessel loses its patency about 30% of the time. In fact, this re-occlusion, known as restenosis, takes place following any sort of coronary arterial intervention, whether balloon angioplasty, atherectomy, stent placement or laser balloon angioplasty, with restenosis rates of approximately 30% at six months (Kuntz et al., " Novel approach to the analysis of restenosis after the use of three new coronary devices," *J. Am. Coll. Cardiol*. 19:1493, 1992). Vascular injury associated with these interventions is understood to initiate a complex cascade of biologic events, such as thrombosis, vascular smooth muscle cell migration and proliferation and production of extracellular matrix (see e.g., lp, et al., J. *Am. Coll. Cardiol*. (1990) 7 : 1667-87; Cassells, W., *Circulation* (1992) 86 : 723-9; Schwartz et al., *J. Am. Coll. Cardiol*. (1992) 20 : 1284-93). Data from experimental and clinical studies have suggested that smooth muscle cell proliferation, in particular, represents a key event that ultimately leads to restenosis in up to 50% of all patients within the first 6 months after the intervention (see, e.g., Hanke et al, *Circ. Res.* (1990) 67 : 651-9; Pickering et al. *J. Clin. Invest*. (1993) 91 : 1469-80). When restenosis occurs, further coronary difficulties can be experienced, including arrhythmia, infarcts and even death.

The systemic administration of antithrombotic and antiproliferative agents in clinical studies, however, has thus far failed to achieve a significant reduction in the incidence of restenosis (Schwartz et al., supra). One explanation for the failure of such trials is that submaximal doses of standard pharmacologic agents have been used because of concerns that serious side effects might result from systemic administration of the required doses. The concept of local, site-specific delivery of pharmacologic and biologic therapies has evolved as a solution (see, e.g., March et al., *Cardio Intervention* (1992) 2:11-26). This concept presumes that higher concentrations of a therapeutic agent maybe achieved directly at the angioplasty site, thus avoiding the toxicity associated with systemic levels of the therapeutic agent.

The inhibition of smooth muscle cell proliferation has been the primary target for local intravascular drug delivery so far. However, the local delivery approach is likely to prove useful for treating a variety of other cardiovascular conditions as well. This includes local delivery of antithrombotic agents, antibiotics, genes, vectors and other biological agents designed to treat or prevent thrombosis, local deposition of angiogenic growth factors designed to promote neovascularization of an ischemic focus and local administration of agents designed to selectively alter vasomotor tone.

Approaches for local, intravascular, site-specific delivery of therapeutic agents have included direct deposition of such agents into the vessel wall through an intravascular delivery system. These intravascular delivery systems generally employ balloon catheters which are easily guided through blood vessels to a region in need of treatment and can then be inflated to contact and dilate the entire surrounding vessel wall. A therapeutic agent can then be delivered to the surrounding vessel wall, for example by diffusion through the balloon or by hydrostatic pressure, as occurs when using a porous balloon catheter. However, clinical use of such catheters is limited by certain practical problems, such as leakage of the solution through side branches of vessels and relatively long latency times of 15 to 30 minutes. Furthermore, the inflation pressure required to accomplish a satisfactory seal between the balloon and the surrounding vessel wall can lead to additional vessel injury proximal and distal to the target site, potentially increasing the proliferative response or creating a nidus for thrombus formation.

Other balloon catheters which have been used for drug delivery to blood vessel walls are drug-coated catheters (e.g., hydrogel catheters). Upon inflation of the balloon in a blood vessel, the therapeutic agent is "pressed" onto or into the surrounding vessel wall. However, a significant disadvantage of this system is that drugs are rapidly washed off the balloon by exposure to the blood-stream during the catheter's passage to the site (see, e.g., Sheriff et al., *J. Am. Coll. Cardiol*. (1993) 21 : 188A). Typically, the balloon must be chaperoned by a protective sheath as the catheter is advanced toward the target vessel. Similarly, the time between sheath removal and balloon inflation must be minimized to avoid premature shedding of the drug into the blood stream at the site prior to balloon inflation.

As an alternative to drug delivery catheters, stents made of polymeric materials have been used for sustained local drug delivery of antithrombotic or antiproliferative drugs, genes or the like. Several approaches have been investigated to achieve continuous drug release from a stent including, for example, seeding the stent with genetically modified endothelial cells to elute agents such as tissue plasminogen activator and coating the stent directly with drugs or with drug-eluding biodegradable polymers. Although these systems can work well, several drawbacks exist. For example, it can be difficult and cumbersome to expand a stent made of polymeric material, and often such stents are costly to manufacture. Additionally, coated metallic stents can be poorly suited for carrying the necessary quantity of therapeutic agent required for effective treatment.

WO 98/23228 discloses in combination the technical features of the pre-characterizing portion of claim 1 below.

WO 97/42910 discloses tubular devices, such as drug infusion catheter/guidewires, which are capable of delivering drugs or other fluids to a specific location within a patient's blood vessel. The guidewire type devices typically comprise a coil spring and a core wire which can be inserted and removed from the lumen of the coil spring. The coated coil contains either side holes or an end hole or a combination thereof in its distal end to enable the drugs or other fluids to be sprayed into the blood vessel.

A need exists for improved devices for local, intravascular delivery of therapeutic agents. A system which delivers a controlled amount of an agent to a blood vessel wall, without creating additional tissue damage or significant inflammatory responses, would satisfy a great need in the art.

### Summary of the Invention

The present invention is defined in claim 1 below. The dependent claims are directed to optional and preferred features.

Accordingly, it is an object of the invention to provide drug delivery devices that provide for local delivery of an agent in a cost-effective, reliable manner.

It is a further object of the invention to provide a local drug delivery system that can carry selected dosages of agent to the site of treatment.

Other objects of the invention will, in part, be obvious, and, in part, be shown from the following description of the systems shown herein.

Devices are disclosed for delivery of one or more therapeutic or diagnostic agents to a body region, such as a blood vessel or a tissue wall. More specifically, the systems disclosed herein include drug delivery devices that comprise a body having a plurality of coils and interstices that occur between the coils and in which a therapeutic or diagnostic agent can be carried.

The drug delivery devices described herein can be guided into a position adjacent to the region to be treated, using conventional techniques. After positioning the device adjacent to the region to be treated, the device can be expanded so that its drug-containing surfaces come into contact with the surrounding tissue. The agent can then pass from the interstices into the surrounding tissue or interstitial fluid.

In one embodiment, the invention provides devices for delivering an agent to a tissue wall of a body lumen. The devices can include a helical body capable of being biased to unwind from a smaller diameter to a larger diameter, and having an exterior surface with the agent disposed thereon. In a wound condition, the device can be sufficiently small to be delivered transvascularly. By unwinding the helical body from the smaller diameter to a larger diameter, the exterior surface of the helical body can be brought into contact with the tissue of the body lumen for delivering the agent thereto. The helical body can be formed of a spring, such as a spring made of inter-woven filaments. Alternatively, the helical body can comprise a spring that has been formed into a plurality of coils to provide the helical body. For example, in one embodiment, the helical body is formed from an elongate, close-pitched spring watch can be looped into a plurality of coils to provide the helical body of the device. In alternate embodiments, the body comprises a tubular body formed into a plurality of coils. The tubular body can have an interior channel with an outer wall with pores disposed therein for allowing delivery of the agent through the outer wall. Alternatively, the outer wall can include a porous membrane that allows for the delivery of the agent through the outer wall. The tubular body can include a coiled wire disposed within the interior channel for biasing the body to unwind from a first diameter to a second, larger diameter. Alternatively, the interior channel can be adapted to receive a pressurized fluid that can cause the body to unwind from a first diameter to a second diameter. In a further embodiment, the helical body can be formed by a plurality of helical bodies that have been brought together to increase the rigidity of the device, and to increase the radial force developed by the device for more securely holding the device body within the body lumen. In other embodiments, an osmotic gradient within an interior lumen of the helical body can attract tissue fluid into the lumen and cause swelling which can unwind the helical body to bring an agent bearing surface into contact with the tissue wall of the body lumen.

In an alternate embodiment, the device can include a section for engaging with a delivery system. This section can act as a tie down for holding the helical body in a wound condition with a diameter sufficiently small to allow for a transvascular delivery to the site to be treated. Additionally, the device can include an optional recapture element, such as an arm or tail, disposed at one end of the device, that allows a catheter to grip a device deployed within a body lumen for the purpose of retracting the device into a catheter, thereby removing the device from the patient.

In another aspect the device can be understood as a device for delivering an agent to biological tissue which comprises a body having a plurality of coils that are optionally capable of unwinding from a smaller diameter to a larger diameter, and that define a plurality of interstices between the coils, wherein the agent is carried within the interstices to be delivered to the biological tissue of the body lumen upon expansion of the body. The tissue can include muscle tissue, including myocardial tissue, oncological tissue, such as tumor mass, and any other suitable physiological and pathological tissue that can be treated by locally delivered agents. The device can be implanted into the tissue to allow the agent to contact the tissue and allow the agent to reach the tissue being treated, which can be the tissue in contact with the device, tissue adjacent to the tissue in contact with the device, tissue that is brought in contact with the agent by delivery of the agent through the blood stream, or any other tissue that can be reached by the agent to promote a therapeutic effect. Accordingly, the devices described herein can treat restenosis, control angiogenesis and vascularization, deliver chemotherapy agents, deliver anesthetics, or perform any other procedure where local delivery of an agent provides a benefit to the patient.

Methods for delivering an agent to a tissue wall of the body lumen for which the present invention is useful, can comprise applying the agent to a surface of a helical body capable of being unwound from a first diameter to a second diameter and having a bias tending to unwind the helical body from the first diameter to the second diameter, disposing the helical body within the body lumen, and unwinding the helical body within the body lumen to allow the agent on the surface of the helical body to contact the tissue wall of the body lumen. After the device is delivered in its wound state to the site of treatment, the device can unwind in response to its internal bias, which can partially drive the device open. Alternatively, the helical body can unwind in response to a fluid being delivered into an interior chamber within the helical body, causing the helical body to open. The fluid can be delivered under pressure, however, alternatively, the pressure build up is in response to a fluid gradient that draws fluid into the interior of the interior chamber.

The disposition of the helical body within a body lumen can be employed in conjunction with the disposition of a stent within a body lumen. The helical body drug delivery device can be disposed within the body lumen and in an subsequent action a stent can be disposed at the same location and coaxial with the helical body thereby providing stent reinforcement of the lumen, as well as local delivery of a therapeutic agent capable of treating restenosis, or treating another condition.

The devices described herein can be manufactured by a process that includes winding an elongate body into a plurality of coaxially aligned coils to form a helical body capable of unwinding from a first diameter to a second diameter and applying to the coils an agent for being carried thereon. In one practice, the elongate body is formed by interweaving a plurality of filaments into a cable. The agent can be carried within the interstices that exist between the filaments. Alternatively, the elongated body can be formed from a close-pitch spring that has been wound into a plurality of coaxially aligned coils. The application of the agent can be achieved by applying a gel material containing the agent to the exterior surface defined by the axially aligned coils of the helical body. Alternatively and optionally, the agent can be delivered into an interior channel that extends through the helical body. In further embodiments, the agent can be carried in a sponge material that fits about the exterior surface of a helical spring body. Optionally, the helical spring body can expand, and in this embodiment, place a force on the sponge that drives the agent from the sponge and into the local tissue.

For restenosis inhibition, a variety of agents can be employed to achieve the desired therapeutic effect. One class of agents inhibits the proliferation of smooth muscle cells. Other agents which treat restenosis and other diseases, including agents which prevent platelet aggregation and adhesion, such as antiplatelets and anticoagulants, can be employed with the devices described herein. In addition, receptor blockers, growth factors and other hormones may be used to limit the normal repair response. The following are groups of particular drugs which can be used to treat vascular disease, such as atherosclerosis and restenosis: anticoagulants, including heparin, hirudin, hirulog, and tissue plasminogen activator, antiinflammatory agents, such as steroids, ibuprofen, aspirin, somatostatin, angiopeptin, and anti-inflammatory peptide 2; cytotoxins, including colchicine, dexamethasone, doxorubicin, methotrexate, and psoen; antibiotics; and enzymes and enzyme inhibitors, including urokinase, 2,4-dinitrophenol, and thiol protease inhibitor and other agents including fibrinogen.

The drug delivery devices described herein can also be used for local, site-specific gene and antisense therapies. In particular, genes, or vectors containing genes, can be delivered which express proteins involved in modulating biologic processes in a body region, such as cell proliferation or matrix production by autocrine means. For example, genes which over express non-secreted growth inhibitors (e.g., tumor suppressor genes) can be transfected into smooth muscle cells present in blood vessel walls to prevent restenosis or proliferation of the cells. Alternatively, to prevent restenosis, genes encoding proteins which cause the death of smooth muscle cells upon exposure to certain drugs can be delivered to blood vessel walls. Genes encoding thymidine kinase can be transfected into vascular smooth muscle cells, rendering the cells vulnerable to gancyclovir. Similarly, factors and agents that promote angiogenesis can be carried by the devices, which can be delivered into the myocardial tissue of the heart. The therapies for which the present invention is useful can be employed to treat ischemic tissue and promote the generation or regeneration of heart tissue.

The devices described herein can also be used to treat blood vessel and arterial blockages. Genes encoding growth factors, such as vascular endothelial growth factor (VEGF), which stimulate new blood vessel growth, can be delivered to the walls of blocked vessels to promote the generation of new vessels which bypass the obstruction.

The drug delivery systems provided by the invention also help avoid the problem of overdosage associated with systemic delivery of drugs, by enabling a controlled amount of a selected therapeutic or diagnostic agent to be directly deposited onto a specific region of tissue. Furthermore, the systems allow for selective delivery of therapeutic or diagnostic agents to vessel walls. The system also solves the problems of drug washoff or leakage.

Overall, the described drug delivery devices provide a safe and effective means for local, site-specific delivery of a wide variety of therapeutic agents to vascular and other body tissues.

### Brief Description of the Figures

The foregoing and other objects and advantages of the invention will be appreciated more fully from the following further description thereof, with reference to the accompanying drawings wherein:
Fig. 1 is an illustration of one embodiment of a drug delivery system for insertion into a body lumen;
Fig. 2 provides an end view of the device of Fig. 1,
Fig. 3 depicts in greater detail one cable suitable for forming the drug delivery device of Fig. 1;
Fig. 4 depicts a method of forming the device of Fig. 1;
Fig. 5 depicts an expanded helical body in a body lumen;
Figs. 6A-6B depict an alternative embodiment of a device for local drug delivery;
Fig. 7 depicts one process for forming the device of Fig. 6A;
Figs. 8 and 9 depict an alternative embodiment of a device for local drug delivery having a portion for being gripped by a delivery system;
Figs. 10 and 11 depict an alternative embodiment of a device for local drug delivery; and
Figs. 12 and 13 depict a delivery system for delivering drug delivery devices.

### Detailed Description of the Illustrated Embodiments

To provide an overall understanding of the invention, certain illustrative embodiments will now be described, including a drug delivery device having a helical body and an exterior surface with a therapeutic agent carried thereon. However, it will be understood by one of ordinary skill in the art that the devices described herein can be adapted and modified to provide systems that can support a vessel wall, deliver a drug to muscle tissue, or any other suitable body tissue, or for any other suitable application. Other changes, additions and modifications can be made to the invention without departing from the scope hereof.

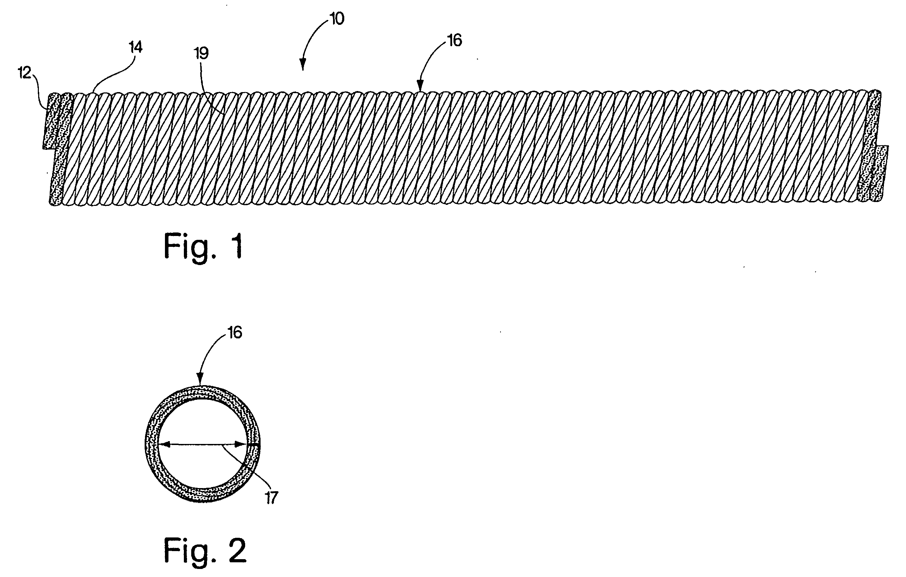

Fig. 1 depicts a drug delivery device 10 that includes a cable 12 that forms a helical body 16 having an exterior surface that can carry a coating 14 containing an agent to be delivered to a tissue wall of a body lumen.

In the depicted embodiment, the helical body 16 of the device 10 is formed as a spring having a closed-pitch, such that adjacent coils of the helical body 16 are in contact, or substantially in contact. In alternative embodiments, the helical body can have an open-pitch wherein adjacent points on separate coils are longitudinally spaced from away from each other. Further embodiments of the helical body 16 can include bodies that are combinations of closed-pitch and open-pitch sections.

As further shown in Fig. 1, the device 10 includes an interstice 19 that is formed as a helical groove on the exterior surface of the body 16 and that extends along the full length of the body 16. The depicted interstice 19 arises from the space that is formed between abutting coils of the helical body 16 and the interstice 19 extends partially into the exterior surface of the helical body 16. In other embodiments, wherein the adjacent coils are non-contiguous, the interstice 19 can extend through the exterior surface of the helical body 16. The interstice 19 provides a space for receiving and carrying an agent, such as a therapeutic agent for treating restenosis. The interstices 19 can increase the amount of coating 14 that can be carried by the exterior surface of the drug delivery device 10 by increasing the surface area of the device 10 relative to a device that has no interstices and by coating spaces in which a coating can reside.

Fig. 2 provides a cross-sectional view of the device 10. Specifically, Fig, 2 shows that the helical body 16 of the device 10 defines an interior lumen 17 that extends longitudinally through the body 16 for the full length of the body 16. As the device 10 can be disposed within a body lumen, such as a coronary artery, it will be understood that the lumen 17 allows for fluid flow through the device 10. Fig. 2, further illustrates that the interior surface of the device 10 is substantially smooth, lacking any elements that extend into the lumen 17. This smooth interior surface is understood to reduce the likelihood of thrombus formation and subsequent flow obstruction. Fig. 2 further illustrates the diameter of the helical body 16. The diameter of the helical body 16 is variable, as the helical body 16 can be coiled, in a spring-like manner, about a longitudinal axis that extends through the helical body 16. This variable diameter facilitates transvascular delivery, as the diameter of the device 10 can be sufficiently reduced to allow the delivery device 10 to fit within and pass through the lumen of an artery or vein of the patient being treated. Accordingly, it will be understood that the delivery device 10 can have a wound state and an unwound state, wherein the delivery device in the wound state has a diameter that is sufficiently small to allow for transvascular delivery and the diameter of the delivery device 10 in the unwound state can be sufficiently large to allow the exterior surface of the helical body 16 to contact the tissue wall of the body lumen in which the device 10 is disposed.

The depicted delivery device 10 is dimensionally adapted for being transvascularly delivered. Specifically, the depicted embodiment is approximately 3 mm in diameter and about 3 cm long. However, the device can be sized for the patient and application, and the diameter can range for example from between 1 mm to about 5 mm, and the length can also vary depending on the application, from .25 cm to about 7 cm. When wound down to a reduced diameter, the device 10 can have a diameter of approximately about .5mm, and when implanted in a body vessel, the device 10 would unwind to a diameter sufficiently large to engage the sidewall of the body vessel. Additionally, the device 10 can unwind to contact the tissue wall with sufficient radial force to secure the device 10 within the vessel and resist downstream movement caused by fluid pressure generated from blood flow.

To facilitate expansion of the delivery device 10 from a first diameter to a second larger diameter, the helical body 16 has a bias that tends to resist being placed in the wound state. To this end, the coils of the depicted helical body 16 are sufficiently resilient to tend to resist rotation or winding, such that rotating the coils around the longitudinal axis places the helical body 16 under tension. This force provides a bias that tends to unwind the helical body 16 and expand the diameter of the device 10 to achieve the diameter of the unwound state. To achieve this resiliency, the helical body 16 can be formed from a material having sufficient elasticity to resist plastic deformation as the body 16 is wound to a reduced diameter. The depicted helical body 16, is formed of a cable 12 made of a plurality of interwoven filaments of elastically deformable material, such as stainless steel 316. The cable 12 can be formed into a plurality of coils to provide the helical body 16. In alternative embodiments, the cable 12 can be formed of other bio-compatible materials, including polymeric materials. Additionally, the device 10 can be formed of bio-absorbable material, thereby allowing the device 10 to be absorbed over time within the body lumen.

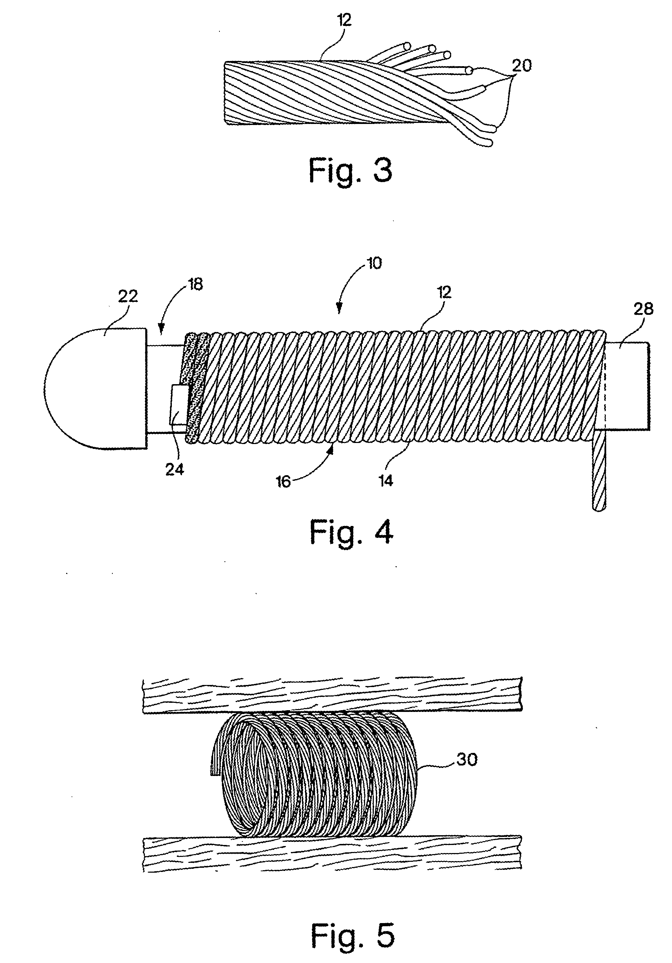

Fig. 3 depicts the individual filaments that are interwoven or intertwined, to form the cable 12 depicted in Fig. 1. As shown by Fig. 3 a plurality of individual filaments 20 are intertwined together to form a cable that is substantially straight and flexible. Each of the depicted filaments 20 can be a strand of a bio-compatible material, such as a metallic or polymeric material, that can be interwoven to form the depicted cable. In one embodiment, the strands 20 are of different materials, including materials containing therapeutic agents for purposes of delivering a drug or a plurality of drugs to the tissue wall of the body lumen, as well as materials selected to achieve a desired flexibility, resiliency, or hoop-strength. It will be apparent to one of ordinary skill in the art that the filaments 20 can be selected to achieve a variety of desired characteristics for the cable 12, and variations in the selection of materials, the methods for interweaving, the additions of adhesives, photo-active compounds or other materials are modifications understood to be within the scope of the invention.

Fig. 4 depicts one process for forming the drug delivery device 10. Specifically, Fig. 4 depicts a mandrel 18 about which the cable 12 is wound. The mandrel 18 includes a handle 22, a chuck 24 and a shaft 28. As shown in Fig. 4, one end of the cable 12 is attached to and held stationary by the chuck 24. The cable 12 can be wound about the shaft 28 of the mandrel 18 causing the cable 12 to coil around the shaft 28 and form into a helical body. The other end of the cable 12 extends freely from the mandrel and can be cut after a selected number of turns are formed on the mandrel to provide a helical body 16 of selected size. The materials selected for forming the cable 12 can impart to the cable 12 a sufficient amount of plastic deformation to allow the cable 12 to be wound on a mandrel, such as the mandrel 28 depicted in Fig. 4, to achieve and to maintain a helical shape. Optionally, the material of the cable 12 can be heated during the process of forming the coils, to facilitate formation of the device 10. In other practices, wherein other materials are employed, such as polymeric materials, treating reagents such as resins, can be applied to the coils to achieve the desired rigidity and resiliency. Once the cable 12 is cut, the chuck 24 can be released so that the wound cable 12 can be slidably removed from the mandrel 18. The drug delivery device 10 can then be placed on an intravascular delivery system that can carry the drug delivery device 10 to the site of deployment within the patient. Although Fig. 4 depicts one process for forming the body of one device according to the invention, it will be understood that other processes can be employed, including processes that involve injection molding, casting processes, laser cutting or etching processes, without departing from the scope of the invention.

Once the body is formed, the coating 14 can be applied to the exterior surface of the device 10, wherein the coating includes at least one agent to be delivered to the vessel wall. The coating 14 can include anti-thrombotic and anti-proliferative agents suitable for reducing the likelihood of restenosis occurring in the vessel. The coating can be applied by dipping the device into a liquid material that will dry to form a coating on the device. In this practice the coating can be formed over the entire exterior and interior of the device, or over a portion of the exterior and interior surfaces. In other processes the coating is formed by brushing a material onto the exterior surface of the device. Other techniques can be employed for coating the device, and the subject matter of the invention is not to be limited to any particular technique or practice. The coating can be placed over the entire exterior of the device, or over selected regions, such as within the groove 19. Additionally, the coating can have alternate thicknesses along the length of the surface and the thickness of the coating can be employed, among other techniques, for controlling the amount of agent delivered to the tissue. The coating can include drug-impregnated gels or polymers capable of being deposited at the endoluminal wall. For the embodiment depicted in Fig. 1, the gel can coat the cable 12, allowing material to travel into the interstices between the coils of the helical body 16 and between the filaments 20 of the cable 12. The agent carried by the coating can be any agent suitable for treating one or more of the conditions that can effect the vascular system, or tissue proximate the vascular system. These agents can include agents for treating restenosis, coronary artery occlusion, myocardial ischemic syndrome, cardiac conduction disturbances or any other condition.

For localized treatment of diseases, the coatings can include agents such as hirudin, argatroban, forskolin, hirulog, heparin, tPA, urokinase, streptokinase, calcium channel blockers, angiotensin converting enzyme inhibitors, fish oil, and growth peptides, thromboxane synthetase inhibitor, serotonin antagonists, HMGCoA, reductase inhibitors, platelet derived growth factors, inflammatory cell factors, platelet aggregation inhibitors, and thrombin inhibitors such as hirudin or its analogs, vapiprost, prostacyclin and prostacyclin analogues, dextran, D-phe-pro-arg-chloromethylketone, dipyridamole, glycoprotein Ilb/Illa platelet membrane receptor antibody, recombinant hirudin, thrombin inhibitor, angiopeptin, angiotensin converting enzyme inhibitors, (such as Captopril, available from Squibb; Cilazapril, available for Hoffman-La Roche; or Lisinopril, available from Merck), colchicine, fibroblast growth factor antagonists, fish oil, omega 3-fatty acid, histamine antagonists, HMG-CoA reductase inhibitor, methotrexate, monoclonal antibodies, nitroprusside, phosphodiesterase inhibitors, prostaglandin inhibitor, seramin, serotonin blockers, steroids, TRANIRAST, thioprotease inhibitors, triazolopyrimidine and other PDGF antagonists, alpha-interferon and genetically engineered epithelial cells, and combinations thereof. The coating material can be bioabsorbable. While the foregoing therapeutic agents have been used to prevent or treat restenosis and thrombosis, they are provided by way of example and are not meant to be limiting, as other therapeutic drugs may be developed which are equally applicable for use with the invention.

Fig. 5 depicts one drug delivery device 30 deployed within a vessel of a patient, such as within a coronary artery. As shown in Fig. 5, the drug delivery device 30 has unwound to an increased diameter that is sufficiently large to engage against the tissue wall of the body lumen. Accordingly, the exterior surface of the drug delivery device 12 is in contact with the tissue wall, thereby contacting the tissue wall with the agent carried in the exterior surface of the drug delivery device 30. In this way, a therapeutic, diagnostic or other type of agent carried on the exterior surface of the delivery device 30 can contact, and be absorbed by, the tissue to be treated. Additionally, the bias of the device 30 provides a force projecting radially outward that tends to secure the device 30 within the body vessel.

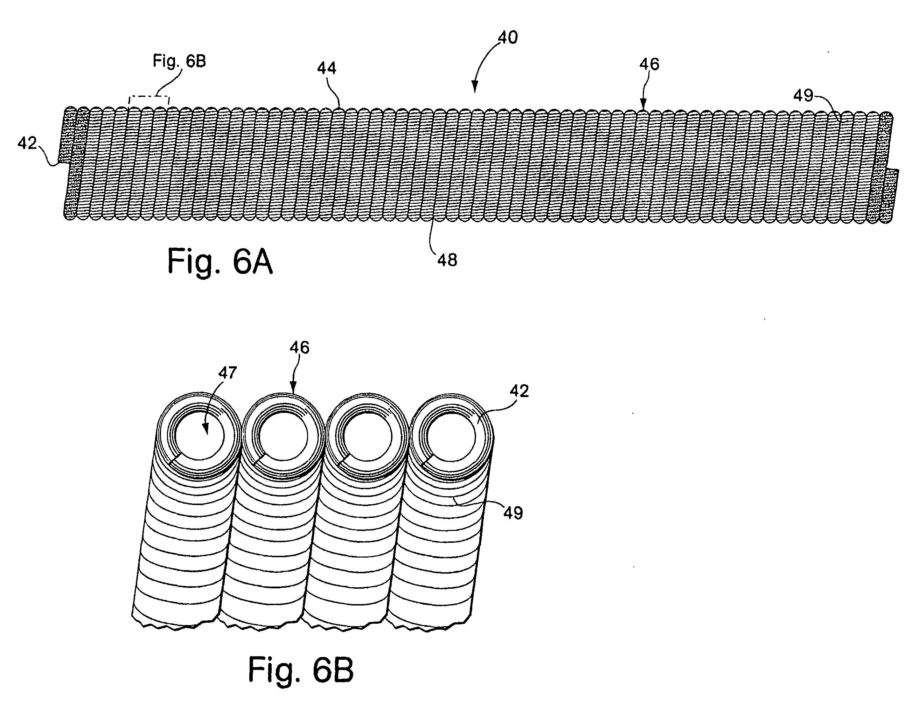

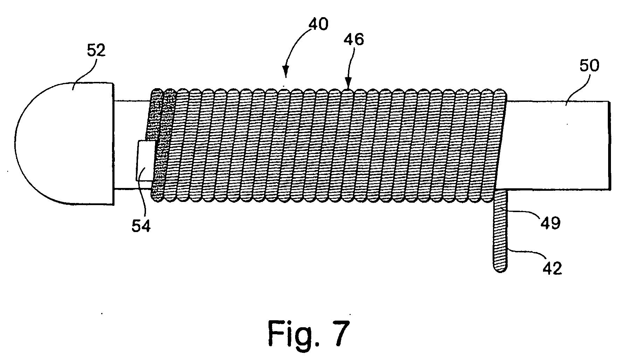

Figs. 6A and 6B depict an alternative embodiment of an implantable device for local drug delivery. Specifically, Fig. 6A depicts a delivery device 40 that includes a helical body 46 formed as a wound spring of bio-compatible material with an exterior surface 44 that can carry the agent to be delivered to the site of treatment. Accordingly, the device 40 can be understood as a compound spring in that it includes a helical, spring-like body 46 that is formed from a smaller spring 42 that has been wound into a plurality of coils. The compound spring structure of the drug delivery device 40 is also shown by Fig. 7 that illustrates in more detail one process for forming the helical body 46 of the device 40. Specifically, Fig. 7 illustrates the spring 42 being wound on a mandrel 50 that mounts to a handle 52 and includes a chuck 54. As shown, the spring 42 can be wound into a plurality of coils that form the helical body 46 of the device 40 in a process that is similar to the process described with reference to Fig. 4. The spring 42 can be formed of a bio-compatible material and generally can be understood as a spring formed of a material that is sufficiently plastically deformable to allow the spring 42 to be wound on the mandrel 50 and shaped into the helical body 46 depicted in Fig. 6. As discussed above, the winding process illustrated by Fig. 7 can be accompanied by a heating process or chemical treatment process that allows the spring 42 to take on and maintain the shape of the helical body 46.

Returning to Fig. 6A it can be seen that the exterior surface 44 of the helical body 46 includes an interstice 48 that curves around the exterior surface 44 and that extends along the full length of the body 46. As discussed above with reference to the embodiment of Fig. 1, the interstice provides a space for receiving a coating that can carry the agent to be delivered to the tissue wall. Fig. 6B illustrates in greater detail and in cross-section one portion of the spring 42. Specifically, Fig. 6b depicts that the spring 42 is formed from a coiled spring wherein adjacent coils of the spring 42 form the depicted interstices 49 that are also capable of carrying an agent to the tissue in watch the device is implantable. Fig. 6B further depicts that the spring 42 includes a lumen 47, which optionally can carry an agent to the tissue. This agent can be the agent applied as a coating to the exterior surface 44 of the device 40, or can be a different material, or a combination of both materials.

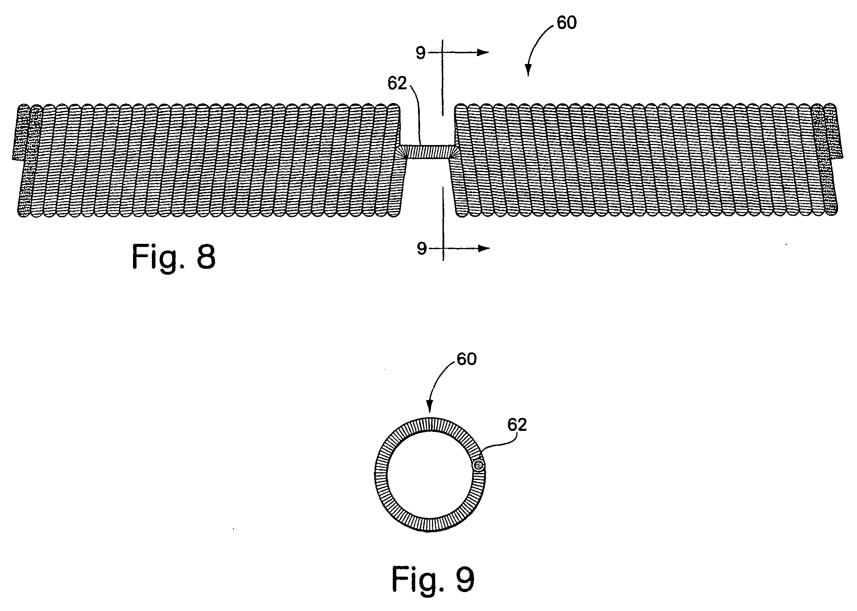

Figs. 8 and 9 depict a drug delivery device 60 that includes a tie down 62 located at the midpoint of the delivery device 60. As shown in Fig. 8, the tie down 62 is formed from a U-shaped bend that is disposed within the helical body of the device 60. The helical body of the device 60 is formed from a coiled spring, similar to the device depicted in Fig. 6. The tie down 62 provides a handle that can be engaged and held by a transvascular delivery system to hold the drug delivery device 60 in a wound condition. Upon release of the U-shaped handle, the drug delivery device will begin unwinding at its central location. This is understood to reduce the likelihood that trauma to the blood vessel will occur by lashing that can arise from the unwinding of the ends of the helical drug delivery device 60. Fig. 9 depicts the cross-section of the device 60, which is similar to that depicted in Fig. 2. It will be understood that although the depicted spring is a compound spring device, such as those shown in Fig. 6A, in other embodiments, the spring can be formed from a cable such as the devices depicted in Fig. 1, or any other suitable device.

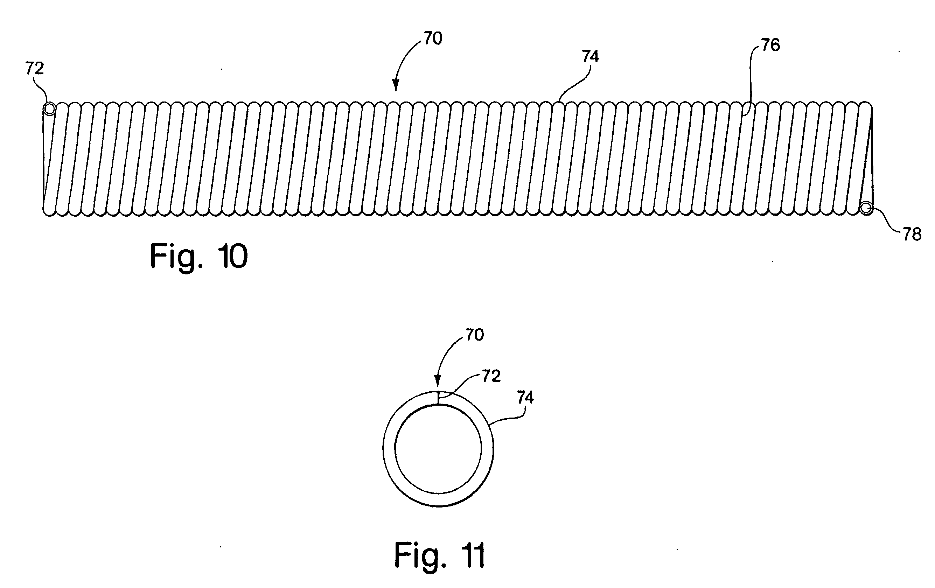

Figs. 10 and 11 depict a further alternative embodiment of a drug delivery device suitable for delivering an agent to a tissue wall of a body lumen. Specifically, Fig. 10 depicts a drug delivery device 70 formed from a cylindrical tube 72 that has been wound into a helical body 74. The helical body 74 can be formed of a tubular rod of bio-compatible material, such as stainless steel 316, and the process employed for forming the tubular rod into the helical body 74 can be similar to the methods described with reference to Fig. 4. The helical body 74 can include a groove 76 that is formed by the space between the adjacent coils of the helical body 74. The exterior surface of the device 70 can be coated with an agent to be delivered to the tissue wall of the body lumen. Any suitable agent can be carried on the exterior surface.

In the depicted embodiment, the cylindrical tube 72 from which the helical body 74 is formed has an interior lumen. Accordingly, the interior lumen provides an interior channel through the coils of the helical body 74. In one embodiment, one end 78 of the helical body is sealed to form a lumen, closed at one end, and thereby adapted for receiving fluid at one end and maintaining fluid therein. Accordingly, a pressurized fluid can be delivered into the interior lumen of the helical body 74. It is understood that delivery of the pressurized fluid into the interior channel of the helical body 74 causes the helix to unwind, thereby expanding the radius of the drug delivery device 70. In one embodiment, the helical body 74 is formed of a porous material that allows the fluid delivered into the interior chamber to pass to the exterior of the device and contact the tissue in which the device is implanted. In this embodiment, the porosity of the wall can be sufficiently limited to prevent rapid release of the delivered fluid and therefore allow sufficient pressure to build within the interior chamber to cause the helical body 74 to unwind. The unwinding of the helical body 74 can be accompanied by a plastic deformation of the device, such that the device can maintain its unwound condition, therefore remaining securely within the body lumen.

In an alternative embodiment, the interior channel of the tubular body carries a resilient wire element that biases the tubular body 74 to tend to unwind the helix of the body 74 to increase the diameter of the drug delivery device 70, thereby allowing contact of the exterior surface of the drug delivery device 70 with the tissue wall of the body lumen being treated. For example, the tubular body can be formed of a polymeric material, or a graft type material, and a resilient coil, such as a coil formed of stainless steel, can be threaded through the interior lumen of the tubular body. The resilient coil provides the tubular body with a bias that tends to unwind the body and expand the body from a smaller diameter to a larger diameter. In this way the internal wire tends to drive the surface of the body into contact with the tissue in which the device is implanted. Optionally, an expandable material can be placed into the interior of the tubular body. The expandable material can contact a body fluid, such as blood, or can respond to tile body heat of the patient, to drive the body open and contact the body against the tissue.

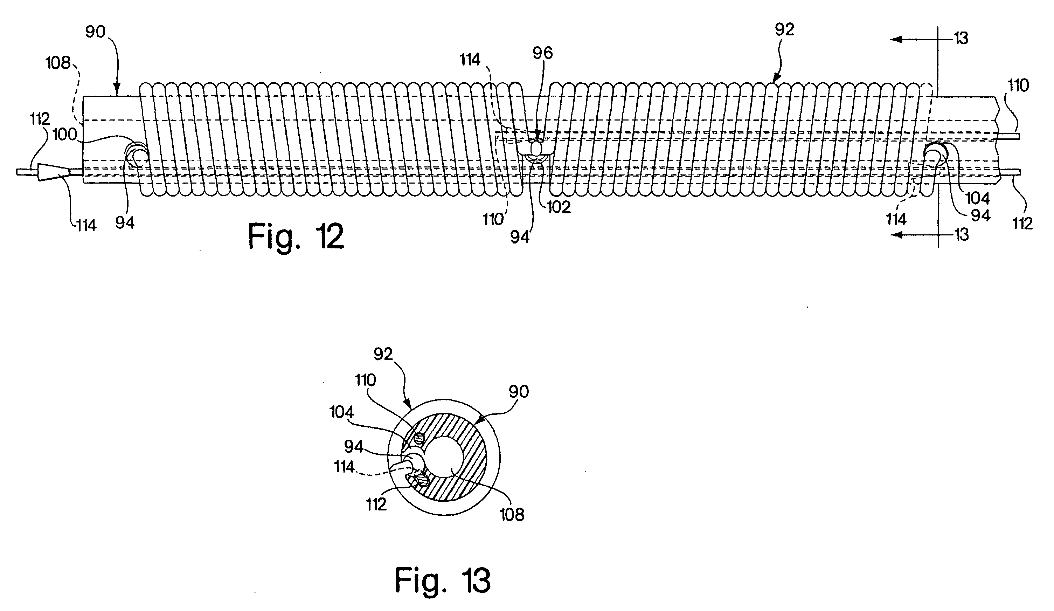

The deployment of the drug delivery systems described above can be accomplished by any suitable system, including those described in U.S. Patent 5,372,600. One illustrative system is depicted in Figs. 12 and 13. Specifically, Fig. 12 depicts a portion of a catheter 90 and a drug delivery device 92 that sits around the periphery of the catheter 90. The catheter 90 can be an elongate tubular body formed of a bio-compatible material and sufficiently long and narrow to allow for transvascular access to the site of treatment. Fig. 12 depicts only the distal portion of the catheter 90, for which in one embodiment the catheter can be 100 to 300 cm in length. The embodiment depicts a drug delivery device 92 that is a compound spring delivery device with an intermediate tie-down, similar to the device depicted in Fig. 8. For the depicted delivery device 92, the ends 94 of the device 92 are terminated in bulbous sections, and the mid-point 96 also has a bulbous section. As illustrated, the bulbous sections are disposed within a plurality of apertures 100,102 and 104 located on the periphery of the catheter 90. The apertures provide openings to a lumen 108 that extends through the interior of the catheter 90. The bulbous sections fit within the apertures and act to retain the drug delivery device 92 on the catheter 90 and in a wound condition. In the depicted embodiment, the bulbous sections fit into the apertures so that the bulbous members catch on the lip formed by the perimeter of the aperture. In this way, the end and mid-sections of the device 92 are prevented from sliding out of the apertures 100, 102, and 104, which act as clasps for securing the device 92 to the body of the catheter 90.

Within the catheter and proximate the lumen 108, two release wires 110 and 112 are disposed within lumens that run along the length of the catheter 90 adjacent to the apertures 100, 102 and 104. The release wires are also shown in Fig. 13. Each of the release wires 110 and 112 has at least one push member 114 that can slide within its respective lumen and pass by one or more of the apertures 100, 102 or 104. The push member 114 can slide past an aperture and push laterally the bulbous section of the drug delivery device away from the lip of the aperture, causing the bulbous member to be released from the aperture. Accordingly, in the depicted embodiment, where the release mechanism includes the separately controllable release wires 110 and 112, the operator can pull the release wires to free the drug delivery device 92 from the catheter 90 and allow the device to unwind and contact the tissue into which the device 92 is being implanted. In one practice, the release wire 110 is operated first to allow the mid-section of-the device 92 to be released. This allows the device to begin unwinding at the midpoint and reduces the force at which the end sections will unwind. This is understood to provide a more gentle deployment of the device 92 and to reduce the likelihood of tissue trauma caused by the unwinding ends of the drug delivery device 92. In a second step, the operator can pull on the release wire 112 to cause the pull member 114, or two pull members 114, to slide past the apertures 100 and 104 and release the end sections of the device 92. By releasing the end sections, the drug delivery device 92 can be fully deployed.

Although the embodiment depicted in Figs. 12 and 13 employs a drug delivery device with bulbous terminations at the end points and midpoints, it will be understood that other embodiments can employ other mechanisms for retaining the drug delivery device on the catheter 90. For example, the ends and mid-sections can employ notched exterior surfaces, wherein the notches are adapted to engage with a tab located adjacent the apertures. Upon moving the push member past the aperture, the grooved section is pushed laterally from the tab, freeing that section of the device. Alternatively, the device 92 can be provided with loops that extend into the apertures and can slidingly receive a release wire, such as the depicted release wires 110 and 112.

Those skilled in the art will know or be able to ascertain using no more than routine experimentation, many equivalents to the embodiments described herein. For example, different materials can be employed for forming the helical body, including composite materials, biodegradable materials, or any other suitable materials. One material can be Nitinol wherein the wound body can expand in response to body temperature of the tissue in which the body is placed. Additionally, the helical body of the device can comprise a plurality of helical bodies, intertwined together. It will also be understood that the systems described herein provide advantages over the prior art including allowing recapture of the drug delivery device, as well as providing a device well suited for use in concert with a stent that supports a weakened or obstructed cardiac artery. Additionally, the drug delivery devices described herein can be employed for delivering drugs to muscle tissue and other types of tissue. Accordingly, it will be understood that the devices described herein can be used in conjunction with delivery systems that can penetrate tissue such as muscle tissue and skin. Similarly, the devices described herein can be employed for delivering agents to the aorta, the iliacs, the urethra, or any internal body lumen.

Accordingly, it will be understood that the invention is not to be limited to the embodiments disclosed herein, but is to be understood from the following claims.

## Claims

1. A device (10, 40, 60, 70) for delivering an agent to a biological tissue, comprising
a helical body (16, 46) comprising a spring (42) formed into a plurality of coils with adjacent turns of the coil spring defining a plurality of mating surfaces disposed adjacent to each other to form interstices (19, 48, 49) therebetween, the spring being capable of being biased to unwind from a first smaller diameter to a second larger diameter, and having an exterior surface (44)
**characterized in that**:
the agent is disposed on said exterior surface, within the interstices occurring between said coils when said helical body has said smaller diameter, wherein the agent is carried within said interstices to be delivered to the biological tissue upon unwinding of the helical body, and the expansion of the helical body to said larger diameter allowing said agent to engage the tissue for delivering the agent thereto.

2. A device according to claim 1, wherein said helical body comprises a spring formed of a cable (12) made of interwoven filaments (20).

3. A device according to claim 2, wherein said interwoven filaments define said interstices.

4. A device according to claim 2, wherein said interwoven filaments include a filament comprising the agent to be delivered.

5. A device according to claim 1, wherein said helical body (74) is formed from a tube (72) which includes an interior channel.

6. A device as claimed in claim 5, wherein the interior channel has a porous outer wall for allowing delivery of the agent through said outer wall.

7. A device according to claim 1, further including a coiled wire disposed within said helical body to provide the bias to said body to unwind from said first diameter to said second diameter.

8. A device according to claim 1, wherein the helical body has a sponge covering.

9. A device according to claim 5, further including a pressurised fluid in the interior channel for biasing said body to unwind from said first diameter to said second diameter.

10. A device according to claim 5, further including an expandable foam body within said interior channel and being adapted for expanding in response to absorbing a body fluid for biasing said body to unwind from said first diameter to said second diameter.

11. A device according to claim 1, wherein said helical body comprises a plurality of helical bodies.

12. A device according to claim 1, further including a section (62) for engaging a delivery system.

13. An apparatus for disposing a drug delivery device within a body lumen, comprising the device of any one of the preceding claims, wherein
said helical body includes a mechanism for cooperating with a delivery system (90) to thereby allow mechanical assisted delivery of the helical body.

14. Apparatus according to claim 13 further comprising
an elongate tubular body having a distal end adapted for carrying the drug delivery device, and
a central latch (114) disposed proximally of said distal end and adapted for engaging a mid-section (96) of the drug delivery device, whereby said central latch secures the mid-section of the drug delivery device to the elongate tubular body for allowing delivery thereof.

15. Apparatus according to claim 14, further comprising
a release element (110, 112) extending through said elongate tubular body and coupled to said latch for operating said central latch to release the mid-section of the drug delivery device.

16. Apparatus according to claim 14, further comprising
a distal latch and a proximal latch each disposed adjacent to said central latch and each capable of engaging the drug delivery device at a respective one of the distal or proximal ends of the drug delivery device.

17. Apparatus according to claim 14, further comprising
a release mechanism capable of being operated to release said distal, central and proximal latches in a sequence in which said central latch is released prior to said distal and proximal latches.

## Patentansprüche

1. Einrichtung (10, 40, 60, 70) zum Abgeben eines Wirkstoffs an ein biologisches Gewebe, mit:
einem spiralförmigen Körper (16, 46), der eine Feder (42) aufweist, die in eine Vielzahl von Wicklungen ausgebildet ist, wobei angrenzende Windungen der Spiralfeder eine Vielzahl von Berührungsflächen definieren, die aneinandergrenzend angeordnet sind, um dazwischen Zwischenräume (19, 48, 49) auszubilden, wobei die Feder vorgespannt werden kann, um sich von einem ersten kleineren Durchmesser auf einen zweiten größeren Durchmesser abzuwickeln, und mit einer äußeren Oberfläche (44), **dadurch gekennzeichnet, dass**:
der Wirkstoff an der äußeren Oberfläche angeordnet ist, innerhalb der Zwischenräume, die zwischen den Wicklungen auftreten, wenn der spiralförmige Körper den kleineren Durchmesser aufweist, wobei der Wirkstoff innerhalb der Zwischenräume getragen wird, um an das biologische Gewebe nach dem Abwickeln des spiralförmigen Körpers abgegeben zu werden, und die Ausdehnung des spiralförmigen Körpers auf den größeren Durchmesser ermöglicht, dass der Wirkstoff in Eingriff mit dem Gewebe gelangt, um den Wirkstoff daran abzugeben.

2. Einrichtung nach Anspruch 1, bei welcher der spiralförmige Körper eine Feder aufweist, die aus einem Kabel (12) ausgebildet ist, das aus verflochtenen Fasern (20) hergestellt ist.

3. Einrichtung nach Anspruch 2, bei der die verflochtenen Fasern die Zwischenräume definieren.

4. Einrichtung nach Anspruch 2, bei der die verflochtenen Fasern eine Faser mit dem abzugebenden Wirkstoff aufweisen.

5. Einrichtung nach Anspruch 1, bei welcher der spiralförmige Körper (74) aus einem Rohr (72) ausgebildet ist, das einen inneren Kanal aufweist.

6. Einrichtung nach Anspruch 5, bei welcher der innere Kanal eine poröse Außenwand aufweist, um die Abgabe des Wirkstoffs durch die Außenwand zu ermöglichen.

7. Einrichtung nach Anspruch 1, ferner mit einem gewickelten Draht, der innerhalb des spiralförmigen Körpers angeordnet ist, um die Vorspannung an dem Körper vorzusehen, damit er sich von dem ersten Durchmesser auf den zweiten Durchmesser abwickelt.

8. Einrichtung nach Anspruch 1, bei welcher der spiralförmige Körper eine Schwamm-Bedeckung aufweist.

9. Einrichtung nach Anspruch 5, ferner mit einem unter Druck stehenden Fluid in dem inneren Kanal, um den Körper vorzuspannen, damit er sich von dem ersten Durchmesser auf den zweiten Durchmesser abwickelt.

10. Einrichtung nach Anspruch 5, ferner mit einem dehnbaren Schaumkörper innerhalb des inneren Kanals und durch den sie dehnbar ist, als Antwort auf ein Absorbieren eines Körperfluids um den Körper vorzuspannen, damit er sich von dem ersten Durchmesser auf den zweiten Durchmesser abwickelt.

11. Einrichtung nach Anspruch 1, bei welcher der spiralförmige Körper eine Vielzahl spiralförmiger Körper aufweist.

12. Einrichtung nach Anspruch 1, ferner mit einem Abschnitt (62), um mit einem Abgabesystem in Eingriff zu gelangen.

13. Vorrichtung zum Anordnen einer Arzneimittel-Abgabeeinrichtung innerhalb eines Körperlumens, mit der Einrichtung nach einem der vorhergehenden Ansprüche, wobei
der spiralförmige Körper einen Mechanismus zum Zusammenwirken mit einem Abgabesystem (90) aufweist, um **dadurch** eine mechanisch unterstützte Abgabe des spiralförmigen Körpers zu ermöglichen.

14. Vorrichtung nach Anspruch 13, ferner mit:
einem länglichen röhrenförmigen Körper mit einem distalen Ende, durch das die Arzneimittel-Abgabeeinrichtung tragbar ist, und
einer zentralen Verriegelung (114), die proximal von dem distalen Ende angeordnet ist, und durch die ein Mittelabschnitt (96) der Arzneimittel-Abgabeeinrichtung in Eingriff bringbar ist, wodurch die zentrale Verriegelung den Mittelabschnitt der Arzneimittel-Abgabeeinrichtung an dem länglichen röhrenförmigen Körper befestigt, um die Abgabe davon zu ermöglichen.

15. Vorrichtung nach Anspruch 14, ferner mit:
einem Löseelement (110, 112), das sich durch den länglichen röhrenförmigen Körper erstreckt und mit der Verriegelung verbunden ist, um die zentrale Verriegelung zu betätigen, um den Mittelabschnitt der Arzneimittel-Abgabeeinrichtung zu lösen.

16. Vorrichtung nach Anspruch 14, ferner mit:
einer distalen Verriegelung und einer proximalen Verriegelung, wobei jede an die zentrale Verriegelung angrenzend angeordnet ist und jede mit der Arzneimittel-Abgabeeinrichtung, an dem entsprechenden distalen oder proximalen Ende der Arzneimittel-Abgabeeinrichtung, in Eingriff gelangen kann.

17. Vorrichtung nach Anspruch 14, ferner mit:
einem Lösemechanismus, der betrieben werden kann, um die distale, zentrale und proximale Verriegelung in einer Reihenfolge zu lösen, bei der die zentrale Verriegelung vor der distalen und der proximalen Verriegelung gelöst wird.

## Revendications

1. Dispositif (10, 40, 60, 70) pour administrer un agent à un tissu biologique, comprenant
un corps hélicoïdal (16, 46) comprenant un ressort (42) formé en une pluralité de bobines avec des enroulements adjacents du ressort en bobine définissant une pluralité de surfaces correspondantes adjacentes les unes aux autres pour former des interstices (19, 48, 49) entre elles, le ressort pouvant être sollicité pour se dérouler d'un premier diamètre plus petit à un second diamètre plus grand, et ayant une surface extérieure (44),
**caractérisé en ce que** :
l'agent est disposé sur ladite surface extérieure, dans les interstices se trouvant entre lesdites bobines lorsque ledit corps hélicoïdal a ledit diamètre plus petit, l'agent étant porté dans lesdits interstices pour être administré au tissu biologique lors du déroulement du corps hélicoïdal, et l'extension du corps hélicoïdal au dit diamètre plus grand permettant au dit agent d'entrer en contact avec le tissu pour lui administrer l'agent.

2. Dispositif selon la revendication 1, dans lequel ledit corps hélicoïdal comprend un ressort formé d'un câble (12) fabriqué en filaments entrelacés (20).

3. Dispositif selon la revendication 2, dans lequel lesdits filaments entrelacés définissent lesdits interstices.

4. Dispositif selon la revendication 2, dans lequel lesdits filaments entrelacés incluent un filament contenant l'agent à administrer.

5. Dispositif selon la revendication 1, dans lequel ledit corps hélicoïdal (74) est formé à partir d'un tube (72) qui inclut un canal intérieur.

6. Dispositif selon la revendication 5, dans lequel le canal intérieur a une paroi externe poreuse pour permettre l'administration de l'agent à travers ladite paroi.

7. Dispositif selon la revendication 1, incluant en outre un fil enroulé disposé dans ledit corps hélicoïdal pour fournir la sollicitation au dit corps pour qu'il se déroule dudit premier diamètre au dit second diamètre.

8. Dispositif selon la revendication 1, dans lequel le corps hélicoïdal a un revêtement spongieux.

9. Dispositif selon la revendication 5, incluant en outre un fluide sous pression dans le canal intérieur pour solliciter ledit corps pour qu'il se déroule depuis ledit premier diamètre vers ledit second diamètre.

10. Dispositif selon la revendication 5, incluant en outre un corps de mousse expansible dans ledit canal intérieur et étant adapté pour s'étendre en réponse à l'absorption d'un liquide organique pour solliciter ledit corps pour qu'il se déroule depuis ledit premier diamètre vers ledit second diamètre.

11. Dispositif selon la revendication 1, dans lequel ledit corps hélicoïdal comprend une pluralité de corps hélicoïdaux.

12. Dispositif selon la revendication 1, incluant en outre une section (62) pour engager un système de mise en place.

13. Appareil pour placer un dispositif d'administration de médicament dans une lumière du corps, comprenant le dispositif de l'une quelconque des revendications précédentes, dans lequel
ledit corps hélicoïdal inclut un mécanisme pour coopérer avec un système de mise en place (90) pour permettre une mise en place assistée mécaniquement du corps hélicoïdal.

14. Appareil selon la revendication 1 3 comprenant en outre
un corps tubulaire allongé ayant une extrémité distale adaptée pour porter le dispositif d'administration de médicament, et
un verrou central (114) placé à proximité de ladite extrémité distale et adapté pour engager une section médiane (96) du dispositif d'administration de médicament, ledit verrou central fixant la section médiane du dispositif d'administration de médicament au corps tubulaire allongé pour permettre sa mise en place.

15. Appareil selon la revendication 14, comprenant en outre
un élément de libération (110, 112) s'étendant à travers ledit corps tubulaire allongé et couplé au dit verrou pour faire fonctionner ledit verrou central pour libérer la section médiane du dispositif d'administration de médicament.

16. Appareil selon la revendication 14, comprenant en outre,
un verrou distal et un verrou proximal placés chacun à côté dudit verrou central et chacun étant capable d'engager le dispositif d'administration de médicament à l'une respective des extrémités distale ou proximale du dispositif d'administration de médicament.

17. Appareil selon la revendication 14, comprenant en outre
un mécanisme de libération pouvant être utilisé pour libérer lesdits verrous distal, central et proximal en une séquence dans laquelle le verrou central est libéré avant lesdits verrous distal et proximal.
